# EUROPÄISCHE PATENTSCHRIFT

(11) **EP 1 424 399 B1**
(45) Veröffentlichungstag und Bekanntmachung des Hinweises auf die Patenterteilung: **11.02.2009**
(21) Anmeldenummer: 03026690.2
(22) Anmeldetag: 20.11.2003
(51) Int. Cl.: C12P 19/36, A61K 31/00, A61K 31/7084, A23L 1/48

(54) **Verfahren zur enzymatischen Reduktion von NAD und NADP**
Process for the enzymatic reduction of NAD and NADP
Procédé pour la réduction enzymatique de NAD et NADP

(30) Priorität: 26.11.2002 DE 10254995
(43) Veröffentlichungstag der Anmeldung: 02.06.2004
(73) Patentinhaber: IEP GmbH, 65203 Wiesbaden (DE)
(72) Erfinder: Gupta, Antje, Dr., 65207 Wiesbaden (DE); Zimmer, Anke, 65205 Wiesbaden (DE)
(74) Vertreter: Zounek, Nikolai

(56) Entgegenhaltungen:
- US-A- 5 444 053
- CHENAULT H.K. AND WHITESIDES G.M.: "Regeneration of Nicotinamide Cofactors for use in Organic synthesis." APPLIED BIOCHEMISTRY AND BIOTECHNOLOGY, Bd. 14, 1987, Seiten 147-197, XP0009027776
- IZUMI Y ET AL: "NADH PRODUCTION FROM NAD+ USING A FORMATE DEHYDROGENASE SYSTEM WITH CELLS OF A METHANOL-UTILIZING BACTERIUM" JOURNAL OF FERMENTATION TECHNOLOGY, TOKYO, JP, Bd. 61, Nr. 2, 1983, Seiten 135-142, XP009025174
- RUSCHIG U. ET AL.: "CO2 Reduction to Formate by NADH Catalysed by Formate Dehydrogenase from Pseudomonas oxalaticus." EUR. J. BIOCHEM., Bd. 70, 1976, Seiten 325-330, XP0009027846

## Beschreibung

Die vorliegende Erfindung betrifft ein Verfahren zur enzymatischen Herstellung von reduziertem Nicotinamid-adenin-dinucleotid (im folgenden NADH) oder reduziertem Nicotinamid-adenin-dinucleotid-phosphat (im folgenden NADPH) durch Formiat-Dehydrogenase katalysierter Reduktion von Nicotinamid-adenin-dinucleotid (im folgenden NAD) oder Nicotinamid-adenin-dinucleotid-phosphat (im folgenden NADP) und einer stabilen NADH- oder NADPH-Zubereitung.

Die Anwendung von NADH oder NADPH lag bisher im wesentlichen in der enzymatischen Analytik und in der medizinischen Diagnostik, wo es in der den verschiedensten Oxidoreduktase Enzymassays zum Einsatz kommt. Seit dem gefunden wurde, dass NADH einen therapeutisch positiven Effekt bei der Behandlung von Müdigkeit, "Jet lag" und bei der Unterstützung der Behandlung von Erkrankungen wie Alzheimer, Parkinson und Depressionen aufweist, hat der Einsatz von NADH breite Anwendung als Nahrungsergänzungsmittel gefunden (US 5,444,053). Dies hat einen ständig steigenden Bedarf von NADH zur Folge.

Die Herstellung von NADH erfolgt bisher durch die Bereitstellung von NAD durch Extraktion aus Bier- oder Bäckerhefe und anschließender Reduktion zu NADH. Es sind eine Reihe von enzymatischen Verfahren zur Reduktion von NAD zu NADH bekannt (H. Chenault et al., Applied Biochemistry and Biotechnology, Band 14, 1987, Seiten 147-197). Bevorzugt kommen dabei die Alkohol-Dehydrogenase, Formiat-Dehydrogenase, Glucose-Dehydrogenase und Glucose-6-phosphat-Dehydrogenase zum Einsatz. Die NADH Herstellung wird im großen Maßstab im wesentlichen mit Alkohol-Dehydrogenase und Ethanol durchgeführt. Dazu wird Ethanol im großen Überschuss eingesetzt und das entstehende flüchtige Acetaldehyd wird kontinuierlich abgeführt. Vorteile dieses Verfahrens sind das preiswerte Ethanol und die in großen Mengen verfügbare Alkohol-Dehydrogenase aus Saccharomyces cerevisiae. Nachteile dieses Verfahrens sind die nicht vollständige Umsetzung von NAD zu NADH und die Entstehung des toxischen Acetaldehyds bei der Synthese.

Die Herstellung von NADPH erfolgt beispielsweise enzymatisch durch NADH-Kinase aus NADH (DE 42 01 930 A1).

Auch die enzymatische Herstellung von NADH mit Formiat-Dehydrogenase ist bekannt. Dabei wird in der Regel Natriumformiat oder Ammoniumformiat eingesetzt. Bei der Reduktion von NAD mit Formiat-Dehydrogenase entsteht Kohlendioxid, das bei saurem bis neutralen pH-Wert aus dem Reaktionsgemisch entweicht. Zur Stabilisierung des entstehenden NADH's und der eingesetzten Formiat-Dehydrogenase sind jedoch pH-Werte von 7,5 bis 8,5 wesentlich besser. Der pH-Wert wird dabei durch Titration mit einer geeigneten Base wie Ammonium, NaOH oder KOH konstant gehalten. Dabei entstehen aus den verwendeten Natriumformiat oder Ammoniumformiat dann entsprechende Natrium- oder Ammoniumcarbonate. Diese Carbonatsalze haben zwar den großen Vorteil, dass sie das NADH stabilisieren, aber sie haben auch den Nachteil, dass sie nur schlecht vom NADH abgetrennt werden können und in großen Mengen bei der Trocknung von NADH durch Einlagerung von Wasser und Ethanol zu Problemen führen.

Die Literaturstelle "NADH production from NAD+ using a Formate Dehydrogenase System with Cells of a Methanol-Utilizing Bacterium", Y. Izumi et al, in "Journal of Fermentation Technology", Tokyo, Japan, Bd. 61, Nr. 2, 1983, Seiten 135-142, beschreibt das Verfahren zur enzymatischen Reduktion von NAD mit Formiat-Dehydrogenase, einem Kaliumphosphatbuffer und Natriumformiat im Reaktionsgemisch. Das Entstehen von Alkalimetallcarbonat wird nicht erwähnt. Der pH-Wert eines eingesetzten Na-carbonatbuffers beträgt 9,2 bis 10.7.

Der Artikel "CO2 Reduction to Formate by NADH Catalysed by Formate Dehydrogena from Pseudomonas oxalaticus", U. Ruschig et al in "EUR. J. BIOCHEM", Bd. 70, 1976, Seiten 325-330 beschreibt eine Direktreduktion von CO₂ zu Formiat durch Produktmengen von NADH. Ein Regenerationssystem für das Reduktionsmittel wird nicht verwendet. Ein System enthaltend Formiat Dehydrogenase, CO₂, HCO₂⁻, NAD, NADH erreicht dabei Gleichgewichtskonzentrationen wie sie mittels thermodynamischen Überlegungen vorausgesagt werden. Die Reaktion kann auch umgekehrt werden, wobei sich das Gleichgewicht verschiebt.

Die Erfindung bezweckt durch Modifikation der Verfahrensbedingungen und Verfahrensschritte die genannten Nachteile zu verbessern. Diese Aufgabe wird erfindungsgemäß gelöst durch den Einsatz einer wässrigen enzymatischen Reduktion von NAD oder NADP mit Formiat-Dehydrogenase und mindestens einem Alkalimetallformiat, wobei vor Isolierung von NADH oder NADPH das gebildete Alkalimetallcarbonat bis auf eine Menge von 0,5 bis 5 Gewichtsprozent aus der Lösung entfernt wird.

Im erfindungsgemäßen Verfahren wird ein Großteil des entstandenen Kohlendioxids beispielsweise durch Calciumionen ausgefällt und ein Anteil von etwa 0,5 % bis 5 % des Kohlendioxids wird durch ein eingesetztes Alkalimetallsalz in Form von beispielsweise Natriumcarbonat in der Reaktionslösung gehalten. Durch Natriumcarbonat wird das gebildete NADH oder NADPH im ganzen Herstellungsverfahren optimal stabilisiert und kann gleichzeitig gut getrocknet werden. Dabei stört Natriumcarbonat nicht, weil es beim Einsatz als Lebensmittelergänzungsstoff auch als Stabilisator zugesetzt wird

Die Erfindung betrifft daher ein Verfahren zur enzymatischen Reduktion von Nicotinamid-adenin-dinucleotid (NAD) oder Nicotinamid-adenin-dinucleotid-phosphat (NADP), das dadurch gekennzeichnet ist, dass man
a) NAD oder NADP mit Formiat-Dehydrogenase und Wasser
b) und mindestens einem Alkalimetallformiat inkubiert,
c) das gebildete Alkalimetallcarbonat durch Ausfällen mit einem Erdalkalisalz bis auf eine Menge von 0,5 bis 5 Gewichtsprozent aus der Lösung entfernt und
d) das entstandene reduzierte Nicotinamid-adenin-dinucleotid (NADH) oder Nicotinamid-adenin-dinucleotid-phosphat (NADPH) isoliert.

Ein weiterer Gegenstand der Erfindung betrifft ein Verfahren zur enzymatischen Reduktion von NAD oder NADP, das dadurch gekennzeichnet ist, dass man
a) NAD oder NADP, Formiat-Dehydrogenase und Wasser
b) mit einem Gemisch bestehend aus mindestens einem Alkalimetallformiat und mindestens einem Erdalkalimetallformiat inkubiert und
c) das gebildete NADH oder NADPH isoliert.

Ein weiterer Gegenstand der Erfindung betrifft ein Verfahren zur enzymatischen Reduktion von NAD oder NADP, das dadurch gekennzeichnet ist, dass man
a) NAD oder NADP, Formiat-Dehydrogenase und Wasser
b) mit einem Gemisch aus Natriumformiat und Calciumformiat inkubiert und
c) das gebildete NADH oder NADPH isoliert.

Unter dem Begriff "Alkalimetallformiat" werden wasserlösliche Salze der Ameisensäure verstanden wie Lithium-, Natrium-, Kalium-, Rubidium- oder Caesiumformiat. Als Alkalimetallformiat kann aber auch Ammoniumformiat eingesetzt werden. Unter dem Begriff "Erdalkalimetallformiat" werden Salze der Ameisensäure verstanden wie Beryllium-, Magnesium-, Calcium-, Strontium- oder Bariumformiat.

Das erfindungsgemäße Verfahren wird beispielsweise in einem geschlossenen Reaktionsgefäß aus Glas oder Metall durchgeführt. Dazu werden die Komponenten NAD oder NADP, Alkalimetallformiat, Erdalkalimetallformiat, Formiat-Dehydrogenase und Wasser einzeln in das Reaktionsgefäß überführt und unter einer Atmosphäre von beispielsweise Stickstoff oder Luft gerührt.

NAD wird in fester Form oder bevorzugt in Wasser gelöst eingesetzt. NAD ist erhältlich durch Extraktion aus Bier- oder Bäckerhefe. Die Konzentration von NAD oder NADP im Syntheseansatz beträgt von 1 % bis 30 %, bevorzugt von 10 % bis 25 %. Diese Prozentwerte beziehen sich auf die Endkonzentration an NADH oder NADPH am Ende der Synthese nach vollständiger Zugabe des NAD oder NADP.

Um einen vollständigen Umsatz des NAD zu NADH, bzw. des NADP zu NADPH, zu gewährleisten, muss das Formiat wie Alkalimetallformiat mindestens äquimolar, bevorzugt aber im Überschuss zum eingesetzten NAD oder NADP vorliegen. Das Formiat kann dabei bis zum zwanzigfachem molaren Überschuss eingesetzt werden, bevorzugt in einem 2 bis 10 fachem molaren Überschuss.

Die Entfernung des gebildeten Alkalimetallcarbonats bis auf eine Menge von 0,5 bis 5 Gewichtsprozent aus der Lösung erfolgt durch Fällung mit einem organischen oder anorganischen Erdalkalisalz, beispielsweise Calciumchlorid, Calciumoxalat, Calciumacetat, Calciummaleat, Magnesiumoxalat, Magnesiumacetat oder Magnesiummaleat.

Die Entfernung des gebildeten Alkalimetallcarbonats kann aber auch durch Zugabe von Erdalkalimetallformiat gleichzeitig mit Alkalimetallformiat oder vor Isolierung von NADH oder NADPH erfolgen.

Das Verhältnis von Erdalkalimetallformiat zu NAD oder NADP beträgt von 0,4 Mol bis 0,9 Mol Erdalkalimetallformiat zu 1 Mol NAD oder NADP, bevorzugt von 0,55 Mol bis 0,75 Mol.

Geeignete NAD-abhängige Formiat-Dehydrogenasen stammen beispielsweise aus Pseudomonas sp. 101, Candida boidinii oder Candida methylica (Gene, Band 162, 1995, Seiten 99-104). Geeignete NADP-abhängige Formiat-Dehydrogenasen sind ebenfalls bekannt (K. Seelbach et al., A novel efficient regenerating method of NADPH using a new formate dehydrogenase, Tetrahedron letters [1996] Band 37, Seiten 1377-1380; oder Tishkov et al., Pilot-scale production and isolation of recombinant NAD and NADP specific formate dehydrogenase, J Biotechnol. Bioeng. [1999] Band 64, Seiten 187-193).

Die einzusetzende Enzymmenge beträgt von etwa 1000 Units bis 1 000 000 Units je kg des verwendeten NAD oder NADP im Reaktionsansatz, bevorzugt von etwa 10 000 bis 100000 Units/kg.

Der Enzymeinheit 1 Unit (U) entspricht dabei der Enzymmenge die benötigt wird um 1 µmol NAD oder NADP je Minute (min) umzusetzen.

Während der Reaktion wird der pH-Wert der Reaktionslösung auf einem Wert von 7 bis 9, bevorzugt von 7,5 bis 8,5, konstant gehalten. Dies erfolgt durch Zugabe einer geeigneten Lauge oder Säure, bevorzugt durch Zugabe von Ammoniumionen.

Die Temperatur beträgt beispielsweise von etwa 10 °C bis 70 °C, bevorzugt von 20 °C bis 35 °C.

Die Reaktionszeit beträgt von 10 Minuten bis 10 Stunden, bevorzugt von 100 Minuten (min) bis 200 min, insbesondere von 120 min bis 150 min.

Anschließend wird das Reaktionsgemisch aufgearbeitet. Nach Abtrennung der unlöslichen Anteile, wie Calciumcarbonat durch Zentrifugation oder Filtration, wird die eingesetzte Formiat-Dehydrogenase durch Ultrafiltration abgetrennt. Die Ultrafiltration kann auch vor Ausfällung des Alkalicarbonats erfolgen. Das Ultrafiltrat wird dann beispielsweise mit Ethanol gefällt. Der lösliche Anteil des Carbonats wie Natriumcarbonat wird dabei zusammen mit dem erhaltenen NADH oder NADPH als Feststoff ausgefällt. Aufgrund der hohen NADH- oder NADPH-Konzentration im Reaktionsansatz sind keine weiteren Aufkonzentrierungsschritte vor der Produktfällung mehr erforderlich.

Die abgetrennte Formiat-Dehydrogenase ist wieder verwendbar.

Nach Ethanolfällung wird das erhaltene Produkt gewaschen und unter reduziertem Druck getrocknet.

Die Erfindung betrifft ferner eine Zubereitung, enthaltend
a) NADH oder NADPH, in einer Menge von 80 bis 95 Gewichtsprozent, bezogen auf die nichtflüchtigen Bestandteile der Zubereitungen und Alkalimetallcarbonat, in einer Menge von 0,5 bis 5 Gewichtsprozent, bezogen auf die nichtflüchtigen Bestandteile der Zubereitung.

Die Erfindung betrifft auch eine Zubereitung, enthaltend
a) NADH oder NADPH, in einer Menge von 80 bis 95 Gewichtsprozent, bezogen auf die nichtflüchtigen Bestandteile der Zubereitungen,
b) Alkalimetallcarbonat, in einer Menge von 0,5 bis 5 Gewichtsprozent, bezogen auf die nichtflüchtigen Bestandteile der Zubereitungen und
c) Wasser, in einer Menge von 3 bis 7 Gewichtsprozent, bezogen auf die nichtflüchtigen Bestandteile der Zubereitung.

Unter dem Begriff "Alkalimetallcarbonat" werden Salze der Kohlensäure verstanden wie Lithium-, Natrium-, Kalium-, Rubidium- oder Caesiumcarbonat oder-hydrogencarbonat.

Die Zubereitung kann auch noch weitere flüchtige und nicht flüchtige Bestandteile enthalten, solange die Menge des NADH's und Alkalimetallcarbonats, bezogen auf die nichtflüchtigen Bestandteile der Zubereitungen, nicht über- oder unterschritten wird.

Die Menge an NADH oder NADPH ist jeweils bezogen auf die nichtflüchtigen Bestandteile der erfindungsgemäßen Zubereitung und sind bevorzugt von 85 Gewichtsprozent bis 95 Gewichtsprozent, insbesondere von 89 Gewichts-% bis 91 Gewichts-%.

Die Menge an Alkalimetallcarbonat ist jeweils bezogen auf die nichtflüchtigen Bestandteile der erfindungsgemäßen Zubereitung und sind bevorzugt von 1 Gewichtsprozent bis 3 Gewichtsprozent, insbesondere von 1,5 Gewichts-% bis 2,5 Gewichts-%.

Die Menge an Wasser ist jeweils bezogen auf die nichtflüchtigen Bestandteile der erfindungsgemäßen Zubereitung und sind bevorzugt von 3 Gewichtsprozent bis 8 Gewichtsprozent, insbesondere von 5 bis 6 Gewichts-%.

Die Herstellung der erfindungsgemäßen Zubereitung erfolgt beispielsweise dadurch, dass man NAD oder NADP, Formiat-Dehydrogenase und Wasser mit einem Gemisch aus Natriumformiat und Calciumformiat inkubiert und das gebildete reduzierte NADH oder NADPH isoliert. Bei der Isolierung von NADH oder NADPH werden unlöslichen Anteile, beispielsweise Calciumcarbonat, durch Zentrifugation oder Filtration abgetrennt, die eingesetzte Formiat-Dehydrogenase wird durch Ultrafiltration abgetrennt und NADH oder NADPH wird aus dem Ultrafiltrat, beispielsweise durch Ethanol, ausgefällt. Das erhaltene Fällungsprodukt wird gewaschen und anschließend getrocknet.

Die erfindungsgemäße Zubereitung weist eine gute Lagerstabilität auf und das Entstehen von Zerfallsprodukten, beispielsweise ADP-Ribose, ADP, AMP oder NAD, wird weitgehend verhindert.

Die erfindungsgemäße Zubereitung enthaltend Wasser, in einer Menge von 3 bis 7 Gewichtsprozent, bezogen auf die nichtflüchtigen Bestandteile der Zubereitung ist besonders gut geeignet für die Verpressung des Materials in Tabletten.

Die Erfindung wird durch die folgenden Beispiele erläutert:

### Beispiel 1

### Synthese von NADH mit Natrium- und Calciumformiat

Der Ansatz enthielt:

| | |
|---|---|
| 150 g NAD (0,225 Mol); | gelöst in 300 ml H₂O |
| 17 g (HCOO)₂Ca (0,132mol) | in Rührgefäß vorgelegt, |
| 20 g (HCOO)Na (0,3 Mol) | in Rührgefäß vorgelegt, |
| 5000 Units Formiat-Dehydrogenase | in Rührgefäß vorgelegt |
| Endvolumen | 750 ml |

Nach 150 min Rühren bei Raumtemperatur mit pH Kontrolle und entsprechender Titration mit NH₃ wurde die Reaktionslösung aufgearbeitet. Die Ausbeute an NADH betrug 120 g Di-Natriumsalz des NADH (70 % der Theorie). Calciumcarbonat wurde durch Zentrifugation abgetrennt und die Abtrennung der Formiat-Dehydrogenase erfolgte durch Ultrafiltration. Nach der Ultrafiltration erhielt man 600 ml Ultrafiltratlösung, die mit 6,9 Liter Ethanol gefällt wurden. Der erhaltene Rückstand wurde unter verminderten Druck getrocknet. Das getrocknete Produkt wies folgende Produktspezifikation auf:

| Bestandteil | Gehalt |
|---|---|
| Formiat/Acetat | 1 % |
| Ethanol | 2,5 % |
| Wasser | 5% |
| Na₂CO₃ | 2 % |
| Reinheit | 97,5 % (2,5 % ADP-Ribose aus Ausgangsmaterial) |
| Gehalt NADH | etwa 89 bis 91 % |

Das auf diese Weise produzierte NADH enthält noch einen Anteil von 1 bis 2 % Na₂CO₃ welches im gesamten Verfahrensablauf stabilisierend wirkt. Dies führt zu einem Produkt, welches frei von Abbauprodukten des NADH ist, gut zu trocknen ist und somit eine optimale Spezifikation für Verpressung und Formulierung für die Anwendung als Nahrungsergänzungsmittel aufweist.

### Beispiel 2

### Synthese von NADH mit Natrium- und Calciumformiat

Der Ansatz enthielt:

| | |
|---|---|
| 50 g NAD (0,074 Mol); | gelöst in 100 ml H₂O |
| 7 g (HCOO)₂Ca (0,054mol) | in Rührgefäß vorgelegt, |
| 7 g (HCOO)Na (0,1 Mol) | in Rührgefäß vorgelegt, |
| 2500 Units Formiat-Dehydrogenase | in Rührgefäß vorgelegt |
| Endvolumen | 250 ml |

Nach 80 min Rühren bei Raumtemperatur mit pH Kontrolle und entsprechender Titration mit NaOH wurde die Reaktionslösung aufgearbeitet. Die Ausbeute an NADH betrug 40 g Di-Natriumsalz des NADH (70 % der Theorie). Calciumcarbonat wurde durch Zentrifugation abgetrennt und die Abtrennung der Formiat-Dehydrogenase erfolgte durch Ultrafiltration . Nach Ultrafiltration wurden 200 ml Reaktionslösung erhalten, die mit 2,3 Liter Ethanol gefällt wurden. Der erhaltene Rückstand wurde unter verminderten Druck getrocknet. Das getrocknete Produkt wies folgende Produktspezifikation auf:

| Bestandteil | Gehalt |
|---|---|
| Formiat/Acetat | 1,5 % |
| Ethanol | 2,4 % |
| Wasser | 6 % |
| Na₂CO₃ | 0,1 % |
| Reinheit | 97,5 % (2,5 % ADP-Ribose aus Ausgangsmaterial) |
| Gehalt NADH | etwa 89 bis 91 % |

## Patentansprüche

1. Verfahren zur enzymatischen Reduktion von Nicotinamid-adenin-dinucleotid (NAD) oder Nicotinamid-adenin-dinucleotid-phosphat (NADP), das **dadurch gekennzeichnet ist, dass** man
a) NAD oder NADP mit Formiat-Dehydrogenase und Wasser
b) und mindestens einem Alkalimetallformiat oder Ammoniumformiat inkubiert,
c) das gebildete Alkalimetallcarbonat oder Ammoniumcarbonat durch Ausfällen mit einem Erdalkalisalz bis auf eine Menge von 0,5 bis 5 Gewichtsprozent aus der Lösung entfernt und
d) das entstandene reduzierte Nicotinamid-adenin-dinucleotid (NADH) oder Nicotinamid-adenin-dinucleotid-phosphat (NADPH) isoliert.

2. Verfahren gemäß Anspruch 1, **dadurch gekennzeichnet ist, dass** man
a) NAD oder NADP, Formiat-Dehydrogenase und Wasser
b) mit einem Gemisch bestehend aus mindestens einem Alkalimetallformiat oder Ammoniumformiat und mindestens einem Erdalkalimetallformiat inkubiert und
c) das gebildete NADH oder NADPH isoliert.

3. Verfahren gemäß Anspruch 1 oder 2, **dadurch gekennzeichnet, dass** als Alkalimetallformiat Lithium-, Natrium-, Kalium-, Rubidium- oder Caesiumformiat eingesetzt wird.

4. Verfahren gemäß einem oder mehreren der Ansprüche 1 bis 3, **dadurch gekennzeichnet, dass** als Erdalkalimetallformiat Beryllium-, Magnesium-, Calcium-, Strontium- oder Bariumformiat eingesetzt wird.

5. Verfahren gemäß einem oder mehreren der Ansprüche 1 bis 4, **dadurch gekennzeichnet, dass** man NAD oder NADP, Formiat-Dehydrogenase und Wasser mit einem Gemisch aus Natriumformiat und Calciumformiat inkubiert und das gebildete reduzierte NADH oder NADPH isoliert.

6. Verfahren gemäß einem oder mehreren der Ansprüche 1 bis 5, **dadurch gekennzeichnet, dass** NAD oder NADP im Syntheseansatz in einer Konzentration von 1 bis 30 Gew.-% eingesetzt wird.

7. Verfahren gemäß Anspruch 6, **dadurch gekennzeichnet, dass** NAD oder NADP im Syntheseansatz in einer Konzentration von 10 bis 25 Gew.-% eingesetzt wird.

8. Verfahren gemäß einem oder mehreren der Ansprüche 1 bis 7, **dadurch gekennzeichnet, dass** man das Alkalimetallformiat mindestens in äquimolarer Menge aber bevorzugt im Überschuss zum eingesetzten NAD oder NADP, einsetzt.

9. Verfahren gemäß Anspruch 8, **dadurch gekennzeichnet, dass** man das Alkalimetallformiat bis zum zwanzigfachem molaren Überschuss zum eingesetzten NAD oder NADP einsetzt.

10. Verfahren gemäß Anspruch 9, **dadurch gekennzeichnet, dass** man Alkaliformiat in einem 2 bis 10 fachem molaren Überschuss zum eingesetzten NAD oder NADP einsetzt.

11. Verfahren gemäß einem oder mehreren der Ansprüche 1 bis 10, **dadurch gekennzeichnet, dass** man die Entfernung des gebildeten Alkalimetallcarbonats durch Fällung mit Calciumchlorid, Calciumoxalat, Calciumacetat, Calciummaleat, Magnesiumoxalat, Magnesiumacetat oder Magnesiummaleat durchführt.

12. Verfahren gemäß einem oder mehreren der Ansprüche 1 bis 9, **dadurch gekennzeichnet, dass** das Verhältnis von Erdalkalimetallformiat zu NAD oder NADP von 0,4 Mol bis 0,9 Mol Erdalkalimetallformiat zu 1 Mol NAD oder NADP beträgt.

13. Verfahren gemäß Anspruch 12, **dadurch gekennzeichnet, dass** das Verhältnis von Erdalkalimetallformiat zu NAD oder NADP 0,55 Mol bis 0,75 Mol Erdalkalimetallformiat zu 1 Mol NAD oder NADP beträgt.

14. Verfahren gemäß einem oder mehreren der Ansprüche 1 bis 13, **dadurch gekennzeichnet, dass** die eingesetzte Enzymmenge von 1000 Units bis 1000000 Units pro kg des verwendeten NAD oder NADP im Reaktionsansatz beträgt.

15. Verfahren gemäß Anspruch 14, **dadurch gekennzeichnet, dass** die eingesetzte Enzymmenge von 10 000 Units bis 100000 Units pro kg des verwendeten NAD oder NADP im Reaktionsansatz beträgt.

16. Verfahren gemäß einem oder mehreren der Ansprüche 1 bis 15, **dadurch gekennzeichnet, dass** der pH-Wert der Reaktionslösung auf einem Wert von 7 bis 9 konstant gehalten wird.

17. Verfahren gemäß Anspruch 16, **dadurch gekennzeichnet, dass** der pH-Wert der Reaktionslösung auf einen Wert von 7,5 bis 8,5 konstant gehalten wird.

18. Verfahren gemäß einem oder mehreren der Ansprüche 1 bis 17, **dadurch gekennzeichnet, dass** die Temperatur 10 °C bis 70 °C beträgt.

19. Verfahren gemäß Anspruch 18, **dadurch gekennzeichnet, dass** die Temperatur 20 °C bis 35 °C, beträgt.

20. Verfahren gemäß einem oder mehreren der Ansprüche 1 bis 19, **dadurch gekennzeichnet, dass** die Reaktionszeit 10 Minuten bis 10 Stunden beträgt.

21. Verfahren gemäß Anspruch 20, **dadurch gekennzeichnet, dass** die Reaktionszeit 100 Minuten (min) bis 200 min beträgt.

22. Verfahren gemäß Anspruch 20, **dadurch gekennzeichnet, dass** die Reaktionszeit 120 min bis 150 min beträgt.

23. Zubereitung, enthaltend
a) NADH oder NADPH, in einer Menge von 80 bis 95 Gewichtsprozent, bezogen auf die nichtflüchtigen Bestandteile der Zubereitung und
b) Alkalimetallcarbonat, in einer Menge von 0,5 bis 5 Gewichtsprozent, bezogen auf die nichtflüchtigen Bestandteile der Zubereitung.

24. Zubereitung gemäß Anspruch 23, **dadurch gekennzeichnet, dass** zusätzlich Wasser in einer Menge von 3 bis 7 Gewichtsprozent, bezogen auf die nichtflüchtigen Bestandteile der Zubereitung, enthalten ist.

25. Zubereitung gemäß den Ansprüche-23 und 24, erhältlich durch ein Verfahren gemäß einem oder mehreren der Ansprüche 1 bis 22, indem während der Isolierung des NADH oder NADPH unlösliche Anteile durch Zentrifugieren oder Filtration abgetrennt, die Formiat-Dehydrogenase durch Ultrafiltration abgetrennt und NADH oder NADPH aus dem Ultrafiltrat ausgefällt, gewaschen und getrocknet wird.

26. Verwendung der Zubereitung gemäß der Ansprüche 23 und 24 als Nahrungsergänzungsmittel bei Müdigkeit, "Jet lag" und bei der Ernährung von an Alzheimer, Parkinson oder Depressionen leidenden Menschen.

## Claims

1. Process for the enzymatic reduction of nicotinamide adenine dinucleotide (NAD) or nicotinamide adenine dinucleotide phosphate (NADP), which process is **characterized in that**
a) NAD or NADP is incubated with formate dehydrogenase and water
b) and at least one alkali metal formate or ammonium formate,
c) the alkali metal carbonate or ammonium carbonate formed is removed from the solution by precipitation with an alkaline earth salt, down to an amount of from 0.5 to 5 per cent by weight, and
d) the reduced nicotinamide adenine dinucleotide (NADH) or nicotinamide adenine dinucleotide phosphate (NADPH) produced is isolated.

2. Process according to Claim 1, **characterized in that**
a) NAD or NADP, formate dehydrogenase and water
b) are incubated with a mixture consisting of at least one alkali metal formate or ammonium formate and at least one alkaline earth metal formate, and
c) the NADH or NADPH formed is isolated.

3. Process according to Claim 1 or 2, **characterized in that** the alkali metal formate used is lithium, sodium, potassium, rubidium or caesium formate.

4. Process according to one or more of Claims 1 to 3, **characterized in that** the alkaline earth metal formate used is beryllium, magnesium, calcium, strontium or barium formate.

5. Process according to one or more of Claims 1 to 4, **characterized in that** NAD or NADP, formate dehydrogenase and water are incubated with a mixture of sodium formate and calcium formate and the reduced NADH or NADPH formed is isolated.

6. Process according to one or more of Claims 1 to 5, **characterized in that** NAD or NADP is used in a concentration of from 1 to 30 % by weight in the synthesis mixture.

7. Process according to Claim 6, **characterized in that** NAD or NADP is used in a concentration of from 10 to 25 % by weight in the synthesis mixture.

8. Process according to one or more of Claims 1 to 7, **characterized in that** at least an equimolar amount, but preferably an excess over the NAD or NADP used, of alkali metal formate is used.

9. Process according to Claim 8, **characterized in that** up to a twentyfold molar excess of alkali metal formate over the NAD or NADP used is used.

10. Process according to Claim 9, **characterized in that** a 2 to 10-fold molar excess of alkali metal formate over the NAD or NADP used is used.

11. Process according to one or more of Claims 1 to 10, **characterized in that** the alkali metal carbonate formed is removed by precipitation with calcium chloride, calcium oxalate, calcium acetate, calcium maleate, magnesium oxalate, magnesium acetate or magnesium maleate.

12. Process according to one or more of Claims 1 to 9, **characterized in that** the ratio of alkaline earth metal formate to NAD or NADP is from 0.4 mol to 0.9 mol of alkaline earth metal formate to 1 mol of NAD or NADP.

13. Process according to Claim 12, **characterized in that** the ratio of alkaline earth metal formate to NAD or NADP is from 0.55 mol to 0.75 mol of alkaline earth metal formate to 1 mol of NAD or NADP.

14. Process according to one or more of Claims 1 to 13, **characterized in that** the amount of enzyme used is from 1000 units to 1 000 000 units per kg of the NAD or NADP used in the reaction mixture.

15. Process according to Claim 14, **characterized in that** the amount of enzyme used is from 10 000 units to 100 000 units per kg of the NAD or NADP used in the reaction mixture.

16. Process according to one or more of Claims 1 to 15, **characterized in that** the pH of the reaction solution is held constant at from pH 7 to pH 9.

17. Process according to Claim 16, **characterized in that** the pH of the reaction solution is held constant at from pH 7.5 to pH 8.5.

18. Process according to one or more of Claims 1 to 17, **characterized in that** the temperature is from 10 °C to 70 °C.

19. Process according to Claim 18, **characterized in that** the temperature is from 20 °C to 35 °C.

20. Process according to one or more of Claims 1 to 19, **characterized in that** the reaction time is from 10 minutes to 10 hours.

21. Process according to Claim 20, **characterized in that** the reaction time is from 100 minutes (min) to 200 min.

22. Process according to Claim 20, **characterized in that** the reaction time is from 120 min to 150 min.

23. Preparation comprising
a) NADH or NADPH in an amount of from 80 to 95 per cent by weight, based on the non-volatile components of the preparation, and
b) alkali metal carbonate in an amount of from 0.5 to 5 per cent by weight, based on the non-volatile components of the preparation.

24. Preparation according to Claim 23, **characterized in that** it additionally contains water in an amount of from 3 to 7 per cent by weight, based on the non-volatile components of the preparation.

25. Preparation according to Claims 23 and 24, obtainable by a method according to one or more of Claims 1 to 22, by removing, during NADH or NADPH isolation, insoluble components by centrifugation or filtration, removing the formate dehydrogenase by ultrafiltration and precipitating from the ultrafiltrate, washing and drying NADH or NADPH.

26. Use of the preparation according to Claims 23 and 24 as food supplement for tiredness, jet lag and for the nutrition of humans suffering from Alzheimer's disease, Parkinson's disease or depression.

## Revendications

1. Procédé pour la réduction enzymatique de nicotinamide-adénine-dinucléotide (NAD) ou de nicotinamide-adénine-dinucléotide-phosphate (NADP), **caractérisé en ce que**
a) on met à incuber du NAD ou du NADP avec de la formiate déshydrogénase et de l'eau,
b) et au moins un formiate de métal alcalin ou du formiate d'ammonium,
c) on élimine de la solution le carbonate de métal alcalin formé ou le carbonate d'ammonium formé, par précipitation avec un sel alcalino-terreux en une proportion de jusqu'à 0,5 à 5 % en poids et
d) on isole le nicotinamide-adénine-dinucléotide réduit (NADH) résultant ou le nicotinamide-adénine-dinucléotide-phosphate réduit (NADPH) résultant.

2. Procédé selon la revendication 1, **caractérisé en ce que**
a) on met à incuber du NAD ou du NADP, de la formiate déshydrogénase et de l'eau
b) avec un mélange constitué d'au moins un formiate de métal alcalin ou de formiate d'ammonium et d'au moins un formiate de métal alcalino-terreux et
c) on isole le NADH ou le NADPH formé.

3. Procédé selon la revendication 1 ou 2, **caractérisé en ce qu'**on utilise en tant de formiate de métal alcalin le formiate de lithium, sodium, potassium, rubidium ou césium.

4. Procédé selon une ou plusieurs des revendications 1 à 3, **caractérisé en ce qu'**on utilise en tant que formiate de métal alcalino-terreux le formiate de béryllium, magnésium, calcium, strontium ou baryum.

5. Procédé selon une ou plusieurs des revendications 1 à 4, **caractérisé en ce qu'**on met à incuber du NAD ou du NADP, de la formiate déshydrogénase et de l'eau avec un mélange de formiate de sodium et formiate de calcium et on isole le NADH réduit formé ou le NADPH réduit formé.

6. Procédé selon une ou plusieurs des revendications 1 à 5, **caractérisé en ce que** le NAD ou le NADP est utilisé dans le mélange initial de synthèse à une concentration de 1 à 30 % en poids.

7. Procédé selon la revendication 6, **caractérisé en ce que** le NAD ou le NADP est utilisé dans le mélange de synthèse à une concentration de 10 à 25 % en poids.

8. Procédé selon une ou plusieurs des revendications 1 à 7, **caractérisé en ce qu'**on utilise le formiate de métal alcalin au moins en quantité équimolaire, mais de préférence en excès par rapport au NAD ou NADP utilisé.

9. Procédé selon la revendication 8, **caractérisé en ce qu'**on utilise le formiate de métal alcalin en un excès jusqu'à 20 fois molaire par rapport au NAD ou NADP utilisé.

10. Procédé selon la revendication 9, **caractérisé en ce qu'**on utilise le formiate de métal alcalin en un excès de 2 à 10 fois molaire par rapport au NAD ou NADP utilisé.

11. Procédé selon une ou plusieurs des revendications 1 à 10, **caractérisé en ce que** l'élimination du carbonate de métal alcalin formé est effectuée par précipitation avec du chlorure de calcium, de l'oxalate de calcium, de l'acétate de calcium, du maléate de calcium, de l'oxalate de magnésium, de l'acétate de magnésium ou du maléate de magnésium.

12. Procédé selon une ou plusieurs des revendications 1 à 9, **caractérisé en ce que** le rapport du formiate de métal alcalino-terreux au NAD ou au NADP va de 0,4 mole à 0,9 mole de formiate de métal alcalino-terreux à 1 mole de NAD ou NADP.

13. Procédé selon la revendication 12, **caractérisé en ce que** le rapport du formiate de métal alcalino-terreux au NAD ou au NADP va de 0,55 mole à 0,75 mole de formiate de métal alcalino-terreux à 1 mole de NAD ou NADP.

14. Procédé selon une ou plusieurs des revendications 1 à 13, **caractérisé en ce que** la quantité d'enzyme utilisée va de 1 000 unités à 1 000 000 unités par kg du NAD ou NADP utilisé dans le mélange réactionnel initial.

15. Procédé selon la revendication 14, **caractérisé en ce que** la quantité d'enzyme utilisée va de 10 000 unités à 100 000 unités par kg du NAD ou NADP utilisé dans le mélange réactionnel initial.

16. Procédé selon une ou plusieurs des revendications 1 à 15, **caractérisé en ce que** le pH de la solution réactionnelle est maintenu constant à une valeur de 7 à 9.

17. Procédé selon la revendication 16, **caractérisé en ce que** le pH de la solution réactionnelle est maintenu constant à une valeur de 7,5 à 8,5.

18. Procédé selon une ou plusieurs des revendications 1 à 17, **caractérisé en ce que** la température est dans la plage de 10 °C à 70 °C.

19. Procédé selon la revendication 18, **caractérisé en ce que** la température est dans la plage de 20 °C à 35 °C.

20. Procédé selon une ou plusieurs des revendications 1 à 19, **caractérisé en ce que** la durée de la réaction va de 10 minutes à 10 heures.

21. Procédé selon la revendication 20, **caractérisé en ce que** la durée de la réaction va de 100 minutes (min) à 200 min.

22. Procédé selon la revendication 20, **caractérisé en ce que** la durée de la réaction va de 120 min à 150 min.

23. Préparation, contenant
a) du NADH ou du NADPH en une proportion de 80 à 95 % en poids, par rapport aux composants non volatils de la préparation et
b) un carbonate de métal alcalin, en une proportion de 0,5 à 5 % en poids, par rapport aux composants non volatils de la préparation.

24. Préparation selon la revendication 23, **caractérisée en ce que** qu'elle contient en outre de l'eau en une proportion de 3 à 7 % en poids, par rapport aux composants non volatils de la préparation.

25. Préparation selon les revendications 23 et 24, pouvant être obtenue par un procédé selon une ou plusieurs des revendications 1 à 22, dans lequel pendant l'isolement du NADH ou NADPH, les fractions insolubles sont séparées par centrifugation ou filtration, la formiate déshydrogénase est séparée par ultrafiltration et le NADH ou le NADPH est précipité à partir du filtrat d'ultrafiltration, lavé et séché.

26. Utilisation de la préparation selon les revendications 23 et 24, en tant que complément nutritionnel dans le cas de fatigue, de "jet lag" (effets du décalage horaire) et dans l'alimentation de sujets souffrants de dépressions, de la maladie de Parkinson ou de la maladie d'Alzheimer.
